(19) Europäisches Patentamt
European Patent Office
Office européen des brevets

(11) Numéro de publication : 0 514 233 A1

(12) **DEMANDE DE BREVET EUROPEEN**

(21) Numéro de dépôt : **92401167.9**

(22) Date de dépôt : **23.04.92**

(51) Int. Cl.[5] : **C07B 37/02,** C07C 69/65, C07C 69/635, C07C 69/675, C07D 307/10, C07D 307/16, C07F 7/08, C07C 67/347, C25B 3/10

(30) Priorité : **23.04.91 FR 9105009**

(43) Date de publication de la demande : **19.11.92 Bulletin 92/47**

(84) Etats contractants désignés : **AT BE CH DE DK ES FR GB GR IT LI LU MC NL PT SE**

(71) Demandeur : **ELECTRICITE DE FRANCE Service National 2, rue Louis Murat F-75008 Paris (FR)**

(72) Inventeur : **Nedelec, Jean-Yves 56, rue de Chalais F-94240 L'Hay-Les-Roses (FR)**
Inventeur : **Khaddouj, Nohair 4, Impasse Victor Schoelcher F-91700 Ste Geneviève-Des-Bois (FR)**
Inventeur : **Paugam, Jean-Paul 45, Boulevard Dubreuil F-91400 Orsay (FR)**

(74) Mandataire : **Koch, Gustave et al Cabinet PLASSERAUD 84, rue d'Amsterdam F-75009 Paris (FR)**

(54) **Procédé électrochimique de préparation de composés organiques par voie radicalaire et composés nouveaux ainsi obtenus.**

(57) Procédé électrochimique de préparation de composés organiques par voie radicalaire mettant en oeuvre un substrat présentant au moins une insaturation carbone-carbone et un réactif, caractérisé par le fait que le réactif est choisi parmi ceux du groupe comprenant :

— d'une part, les dérivés organiques halogénés oxydables comportant un atome d'hydrogène énolisable de formule

$$XCHA_2$$

dans laquelle :

— X est un atome d'halogène du groupe Cl, Br, I et

— A est un radical du groupe -CN, -COR′ et -$CO_2R′$ avec R′ représentant un radical alkyle fonctionnalisé ou non, linéaire ou ramifié, notamment les radicaux méthyle, éthyle et tertio-butyle,

— d'autre part, les dérivés organiques halogénés non oxydables ne comportant pas d'hydrogène énolisable et répondant aux formules :

$$X_2CY_2 \text{ et}$$
$$X_2CA_2$$

dans lesquelles X et A ont les significations indiquées ci-dessus et Y représente un atome de chlore ou de fluor, étant entendu que ces dérivés organiques halogénés non oxydables sont mis en oeuvre en présence d'un composé à méthylène actif.

EP 0 514 233 A1

L'invention a pour objet un procédé électrochimique de préparation de composés organiques par voie radicalaire, ledit procédé étant du genre de ceux dans lesquels on forme, par réaction radicalaire, une liaison carbone-carbone entre un composé fonctionnalisé et un composé organique présentant au moins une insaturation carbone-carbone.

L'invention vise également ceux des composés organiques halogénés ainsi obtenus qui sont nouveaux.

Les composés en question connus ou nouveaux, outre leur intérêt propre, constituent des matières premières précieuses pour la préparation de nombreux produits notamment du domaine des industries de la pharmacie, de la parfumerie et des cosmétiques et de l'agrochimie.

Il est connu, dans les procédés du genre en question, d'amorcer la réaction radicalaire par mise en oeuvre d'un métal à degré d'oxydation élevé ou d'un peroxyde; les inconvénients de ces méthodes sont:

- que le métal à degré d'oxydation élevé doit être utilisé en large excès (2 à 3 équivalents par équivalent de substrat à transformer), ce qui constitue un inconvénient majeur dans la mesure où, d'une part, la forme oxydée est souvent difficile d'accès et instable et où, d'autre part, la forme réduite résultant de la transformation chimique n'est pas facilement récupérable, ce qui entraîne des problèmes de pollution;
- qu'avec le peroxyde, les réactions doivent être conduites à température élevée (>100°C), la polymérisation du substrat insaturé n'étant évitée par ailleurs que par l'usage d'un excès du composé fonctionnalisé mis en oeuvre.

Il a déjà été proposé de régénérer par voie électrochimique in situ le métal à degré d'oxydation élevé lorsqu'on a recours à ce moyen pour l'amorçage des réactions radicalaires (cf. Nishigushi et al., Tetrahedron 1991, vol. 47, pages 831-840); le procédé décrit par Nishigushi est mis en oeuvre dans une cellule divisée; grâce à ce procédé, il n'est plus nécessaire d'avoir recours à un gros excès de métal à degré d'oxydation élevé mais la consommation électrique est très importante, à savoir au moins 2 F/mol.

L'invention a pour but, surtout, de remédier aux inconvénients de l'art antérieur et de fournir un procédé du genre en question dont la simplicité, l'efficacité et le bilan économique le rendent particulièrement intéressant pour l'industrie.

Et il est du mérite des Inventeurs d'avoir trouvé que ce but pouvait être atteint de manière surprenante et inattendue dès lors que l'on a recours à un composé fonctionnalisé choisi parmi ceux du groupe comprenant les dérivés organiques halogénés oxydables comportant un hydrogène énolisable et les dérivés organiques halogénés non oxydables dépourvus d'hydrogène énolisable mais mis en oeuvre en combinaison avec un composé à méthylène actif.

En conséquence, le procédé conforme à l'invention --suivant lequel on fait réagir un composé organique appelé "substrat" et présentant au moins une insaturation carbone-carbone avec un composé organique fonctionnalisé appelé "réactif" pour relier le substrat au réactif par une liaison carbone-carbone qui est créée par une réaction radicalaire amorcée par un métal appelé "médiateur" porté électrochimiquement et in situ à un degré d'oxydation élevé au sein d'un solvant polaire qui est compatible avec le potentiel d'oxydation du médiateur, qui contient un électrolyte-support et dans lequel plongent deux électrodes entre lesquelles on établit une différence de potentiel suffisante pour assurer l'oxydation électrochimique du médiateur-- est caractérisé par le fait que le réactif est choisi parmi ceux du groupe comprenant:

- d'une part, les dérivés organiques halogénés oxydables comportant un atome d'hydrogène énolisable de formule

$$XCHA_2$$

dans laquelle:

  - X est un atome d'halogène du groupe Cl, Br, I et
  - A est un radical du groupe -CN, -COR′ et -$CO_2$R′ avec R′ représentant un radical alkyle fonctionnalisé ou non, linéaire ou ramifié, notamment les radicaux méthyle, éthyle et tertio-butyle,

- d'autre part, les dérivés organiques halogénés non oxydables ne comportant pas d'hydrogène énolisable et répondant aux formules:

$$X_2CY_2 \text{ et}$$
$$X_2CA_2$$

dans lesquelles X et A ont les significations indiquées ci-dessus et Y représente un atome de chlore ou de fluor, étant entendu que ces dérivés organiques halogénés non oxydables sont mis en oeuvre en présence d'un composé à méthylène actif choisi dans le groupe comprenant ceux des formules:

$$CNCH_2CO_2R'$$
$$MeCOCH_2CO_2R'$$
$$MeCOCH_2COMe$$

dans lesquelles Me est le radical méthyle et R′ a les significations indiquées plus haut,

ce grâce à quoi il s'établit un processus en chaîne dont il sera plus explicitement question ci-dessous et qui permet d'abaisser substantiellement la consommation électrique.

Lorsque le réactif répond à la formule $XCHA_2$, le substrat à la formule R″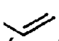 et que le médiateur est représenté par $M^{m+}$, le schéma réactionnel est celui indiqué ci-dessous, R″ représentant un radical aryle fonctionnalisé ou non, un radical alkyle fonctionnalisé ou non, rectiligne ou ramifié, aralkyle fonctionnalisé ou non avec alkyle rectiligne ou ramifié. A la cathode, il se produit la réaction:

$$H^+ + e \rightarrow 1/2\ H_2$$

A l'anode, il se produit la réaction:

$$M^{m+} - ne \rightarrow M^{(n+m)+}$$

Le métal oxydé réagit sur le réactif suivant la réaction:

$$M^{(n+m)+} + nXCHA_2 \rightarrow n(XCA_2)\cdot + nH^+ + M^{m+}$$

$$(XCA_2)\cdot + HXCA_2 \rightarrow X_2CA_2 + (HCA_2)\cdot$$

le symbole "·" identifiant une forme radicalaire.

Et enfin il s'établit le processus en chaîne suivant au sein du solvant:

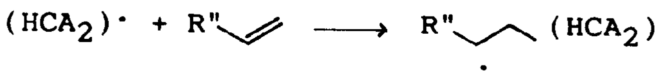 (1)

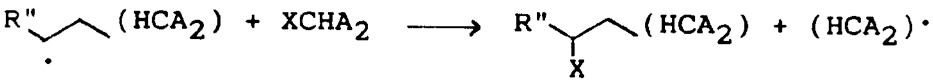 (2)

le radical ainsi formé $(HCA_2)\cdot$ réagissant de nouveau suivant (1) et ainsi de suite.

Lorsque le réactif répond à l'une des formules

$$X_2CY_2 \text{ et } X_2CA_2,$$

c'est-à-dire dans le cas où la réaction doit être effectuée en présence d'un composé à méthylène actif, encore appelé "co-médiateur", le schéma réactionnel est celui indiqué ci-après, étant entendu que le médiateur est de nouveau représenté par $M^{m+}$ et le substrat par R″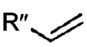 ; dans ce cas particulier, le réactif est par exemple $X_2CA_2$ et le co-médiateur $CNCH_2CO_2R'$.

Il se produit alors, à la cathode, la même réaction que dans le cas précédent.

Par contre, à l'anode, il se produit les trois réactions suivantes:

$$M^{m+} - n\ e \rightarrow M^{(n+m)+}$$

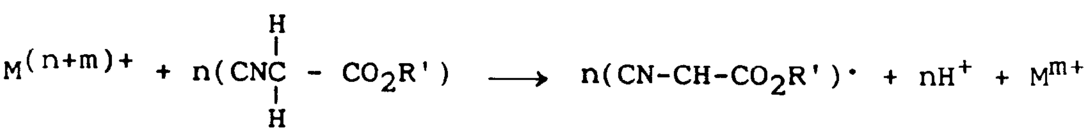

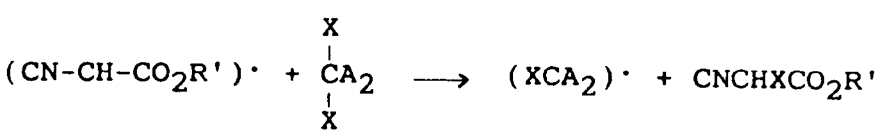

Enfin, comme dans le premier cas, il s'établit au sein du solvant un processus en chaîne indiqué ci-après:

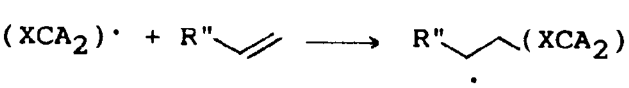 (3)

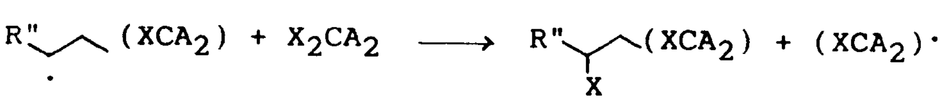 (4)

et ainsi de suite.

Les solvants utilisés dans le procédé conforme à l'invention sont ceux utilisés habituellement en électrooxydation et compatibles avec le potentiel d'oxydation du médiateur; ils peuvent être choisis dans le groupe comprenant l'acide acétique, le méthanol et l'acétonitrile; ils peuvent également être constitués par un mélange d'un solvant polaire protique et d'un solvant polaire aprotique, par exemple l'acide acétique et l'acétate d'éthyle ou l'acide acétique et l'acétonitrile; les solvants préférés sont l'acide acétique et le méthanol.

L'électrolyte support dont le rôle est de rendre le milieu réactionnel conducteur est avantageusement constitué par un acétate et peut être choisi dans le groupe comprenant les acétates de potassium, de sodium et d'ammonium; il est présent à une concentration d'environ 0,1 à environ 1, de préférence de 0,4 à 0,8 mol/litre; il intervient, en fonction de sa concentration, au niveau de la vitesse globale de la réaction.

Le substrat insaturé peut être choisi parmi:

- les alcènes ou cyclo-alcènes fonctionnalisés ou non,
- les diènes conjugués ou non,
- les alcynes fonctionnalisés ou non.

Les concentrations en substrat et en réactif sont toutes deux comprises entre environ 0,1 et environ 1,5, de préférence entre 0,4 et 1 mol/litre.

Le rapport des concentrations en substrat et en réactif est compris entre environ 0,5 et environ 5 et, de préférence, entre 0,1 et 2.

Le médiateur constitué par le métal à degré d'oxydation élevé, de préférence le manganèse (III), le cobalt (III), le cérium (IV) et le fer (III), est mis en oeuvre à partir du sel de Mn(II), de Co(II), de Ce(III) et de Fe(II), de préférence d'un acétate, notamment l'acétate de manganèse.

La concentration en médiateur ou plus exactement en sel du métal à degré d'oxydation élevé est d'environ 0,01 à environ 0,1, de préférence comprise entre 0,02 et 0,04 mol/litre.

Le procédé est mis en oeuvre à une température allant de 0°C à la température d'ébullition du solvant; de préférence, on travaille à une température d'environ 40 à environ 60°C pour assurer une bonne homogénéité du milieu.

Le procédé est généralement mis en oeuvre à la pression atmosphérique; toutefois, dans le cas de l'utilisation d'un réactif gazeux, on préfère maintenir une légère surpression au-dessus du milieu réactionnel.

Il n'est pas nécessaire de travailler sous atmosphère contrôlée; on peut toutefois travailler sous atmosphère constituée du réactif lorsque celui-ci est gazeux.

Le procédé est mis en oeuvre dans une cellule compartimentée ou non; pour des raisons de simplicité, on a recours à une cellule non compartimentée, et ceci sans perte d'efficacité.

L'anode est constituée en un matériau électrochimiquement inerte, tel que par exemple de la fibre ou mousse de carbone; elle peut également être constituée par une plaque ou grille, de préférence cylindrique, de platine ou de titane.

La cathode est constituée en un matériau chimiquement et électrochimiquement inerte, par exemple en fibre ou mousse de carbone, platine ou acier inoxydable.

L'invention sera encore mieux comprise à l'aide des exemples qui suivent et qui ne sont pas limitatifs mais se rapportent à des modes de réalisation particuliers et, le cas échéant, avantageux.

Ils ont été réalisés à l'aide d'une cellule non compartimentée dont le volume utile est de 50 $cm^3$; l'anode a une surface de travail de 20 $cm^2$ environ et la cathode une surface utile de 0,1 $cm^2$ environ, l'anode étant disposée concentriquement autour de la cathode.

Dans ces exemples, le solvant qui est choisi parmi ceux du groupe comprenant AcOH, MeOH, $CH_3CN$, AcOEt et leurs mélanges, peut être utilisé sans purification. L'électrolyte support est présent à la concentration de 0,8 M, sauf indication contraire. Le médiateur, le plus souvent l'acétate de manganèse, est généralement à la concentration de 0,04 mol/l; le co-médiateur, s'il est présent, est généralement à la concentration de 0,04 mol/l. Le volume de la solution est de 50 $cm^3$. La cellule est plongée dans un bain thermostatique réglé à la température choisie. L'électrolyse est conduite à intensité constante, généralement de 0,1 A, et poursuivie jusqu'à disparition de celui des deux partenaires de la réaction, à savoir le substrat ou le réactif, qui est minoritaire. L'évolution de la réaction est suivie par dosage chromatographique en phase gazeuse des réactifs et des produits obtenus.

Une fois l'électrolyse terminée, on évapore le solvant. Le résidu est repris par un solvant organique, par exemple le diéthyléther, le $CH_2Cl_2$ ou le pentane et lavé à l'eau. Après séchage et évaporation du solvant, les produits obtenus sont séparés et isolés par chromatographie puis identifiés par les méthodes classiques d'analyse organique, à savoir l'analyse élémentaire, la RMN, les analyses SM et IR.

## EXEMPLE 1

**Préparatlon du bromo-4-méthoxycarbonyl-2-nonanoate de méthyle.**

Le composé identifié ci-dessus est préparé par addition du monobromomalonate de diméthyle sur l'heptène-1.

L'appareillage utilisé est constitué par la cellule décrite plus haut.

Cette cellule comporte, d'une part, une anode en fibre de carbone et, d'autre part, une cathode en acier inoxydable, les dimensions étant celles indiquées ci-dessus.

On introduit dans cette cellule 50 $cm^3$ de solvant constitué par de l'acide acétique.

On dissout dans le solvant 4 g d'électrolyte support constitué par l'acétate de potasium, ce qui conduit à une concentration molaire de 0,8 $mol.l^{-1}$.

Le substrat est constitué par de l'heptène-1 et il est introduit dans le milieu réactionnel à raison de 5,6 $cm^3$, ce qui conduit à une concentration molaire de 0,8 $mol.l^{-1}$.

Le réactif est constitué par le bromomalonate de diméthyle et il est introduit dans le milieu réactionnel à raison de 4,2 g, ce qui correspond à une concentration molaire de 0,4 $mol.l^{-1}$.

Le médiateur est constitué par l'acétate de manganèse qui est introduit à raison de 0,5 g dans le milieu réactionnel, ce qui correspond à une concentration molaire de 0,04 $mol.l^{-1}$.

La température du milieu réactionnel est maintenue à 60°C en plongeant la cellule dans un bain thermostatique. Le milieu réactionnel est agité à l'aide d'un barreau magnétique.

On impose entre les deux électrodes une intensité de 0,1 A pendant la durée nécessaire à la consommation totale de l'halogénure; cette durée est de 4 h 50 min; la consommation d'électricité correspondante est de 0, 9 F/mol calculée par rapport à la concentration du réactif.

Le solvant est évaporé et le résidu repris par un solvant organique constitué par du diéthyléther; on procède à un lavage à l'eau.

Après séchage et évaporation du solvant, les produits obtenus sont séparés et isolés par chromatographie puis identifiés par RMN, SM et analyse élémentaire.

On obtient 4,6 g de bromo-4-méthoxycarbonyl-2-nonanoate de méthyle, ce qui correspond à un rendement de 78% en produit isolé par rapport au réactif initial.

Le taux de conversion du réactif est de 100.

Le bromo-4-éthoxycarbonyl-2-nonanoate d'éthyle a été obtenu dans les mêmes conditions à partir du mono-bromomalonate de diéthyle également avec un rendement de 78% en produit isolé par rapport au réactif initial.

## EXEMPLES 2 à 6

En procédant de la manière indiquée à l'exemple 1, on fait réagir le bromomalonate de diméthyle successivement sur cinq substrats correspondant respectivement aux exemples 2 à 6; l'identification de ces substrats, des produits obtenus, de la consommation d'électricité, du rendement et du taux de conversion du réactif sont réunis dans le tableau I indiqué ci-après.

TABLEAU I

| Ex. No. | Réactif ($mol.l^{-1}$) | Substrat ($mol.l^{-1}$) | Médiateur ($mol.l^{-1}$) | Co-médiateur ($mol.l^{-1}$) | Solvant | Electrolyte support ($mol.l^{-1}$) | Temp. °C | Produit final | a F/mol | b ρ % | c t % |
|---|---|---|---|---|---|---|---|---|---|---|---|
| 2 | $BrCH(CO_2Me)_2$ (0,4) | 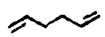 (0,8) | $Mn(OAc)_2$ (0,04) | - | AcOH | AcOK (0,8) | 60 | 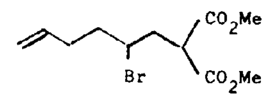 | 0,7 | 75 | 100 |
| 3 | $BrCH(CO_2Me)_2$ (0,4) | 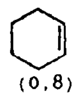 (0,8) | $Mn(OAc)_2$ (0,04) | - | AcOH | AcOK (0,8) | 60 | 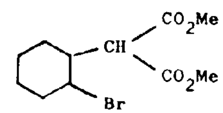 | 2 | 42 | 80 |
| 4 | $BrCH(CO_2Me)_2$ (0,4) | 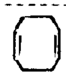 (0,8) | $Mn(OAc)_2$ (0,04) | - | AcOH | AcOK (0,8) | 60 | 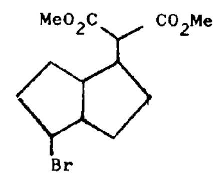 | 0,6 | 85 | 100 |
| 5 | $BrCH(CO_2Me)_2$ (0,4) | 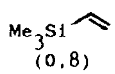 (0,8) | $Mn(OAc)_2$ (0,04) | - | AcOH | AcOK (0,8) | 60 | 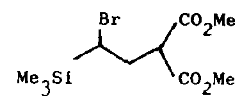 | 1 | 40 | 80 |
| 6 | $BrCH(CO_2Me)_2$ (0,4) | 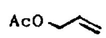 (0,8) | $Mn(OAc)_2$ (0,04) | - | AcOH | AcOK (0,8) | 60 | 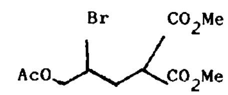 | 1 | 80 | 100 |

(a) Electricité consommée calculée par rapport à la concentration du réactif

(b) Rendement en produit isolé par rapport au réactif

(c) Taux de conversion du réactif

## EXEMPLE 7

**Préparation du dibromo-2,4-méthoxycarbonyl-2-nonanoate de méthyle.**

En reprenant les conditions générales indiquées à l'exemple 1, on prépare le composé identifié ci-dessus par addition du dibromomalonate de diméthyle à l'heptène-1.

La quantité de dibromomalonate de diméthyle est de 5,8 g, ce qui correspond de nouveau à une concentration molaire de 0,4 comme à l'exemple 1.

Le médiateur est constitué, comme dans l'exemple 1, par l'acétate de manganèse qui est introduit à raison de 0,5 g dans le milieu réactionnel, ce qui correspond à une concentration molaire de 0,04 mol.$l^{-1}$.

Dans cette réaction, le co-médiateur est constitué par du cyanoacétate de méthyle ajouté en une quantité de 0,2 g, ce qui correspond à une concentration molaire de 0,04.

La température du milieu réactionnel est maintenue à 60°C.

La consommation d'électricité est de 0,06 F/mol calculée par rapport à la concentration initiale du réactif.

On obtient 5,4 g de dibromo-2,4-méthoxycarbonyl-2-nonanoate de méthyle, ce qui correspond à un rendement de 70**%** en produit isolé par rapport au réactif initial.

Le taux de conversion du réactif est de 100.

## EXEMPLES 8 à 17

Dans ces exemples qui concernent tous l'addition du dibromomalonate de diméthyle sur l'heptène-1, on étudie l'influence sur le déroulement de la réaction des divers paramètres constitués par la nature du solvant, la température, la concentration et la nature de l'électrolyte support, et la concentration du support et du réactif.

Le médiateur et le co-médiateur mis en oeuvre sont ceux de l'exemple 7 aux mêmes concentrations.

On a réuni dans le tableau II ci-après ces différents facteurs ainsi que la consommation d'électricité, le rendement et le taux de conversion du réactif.

TABLEAU II

| Ex. No. | Réactif (mol.l$^{-1}$) | Substrat (mol.l$^{-1}$) | Médiateur (mol.l$^{-1}$) | Co-médiateur (mol.l$^{-1}$) | Solvant | Electrolyte support (mol.l$^{-1}$) | Temp. °C | Produit final | a F/mol | b ρ % | c t % |
|---|---|---|---|---|---|---|---|---|---|---|---|
| 8 | $Br_2C(CO_2Me)_2$ (0,4) | 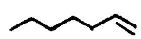 (0,8) | $Mn(OAc)_2$ (0,04) | $CNCH_2CO_2Me$ (0,04) | AcOH | AcOK (0,4) | 60 | 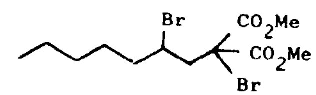 | 0,1 | (46) | 100 |
| 9 | $Br_2C(CO_2Me)_2$ (0,4) | 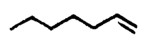 (0,4) | $Mn(OAc)_2$ (0,04) | $CNCH_2CO_2Me$ (0,04) | AcOH | AcOK (0,4) | 60 | 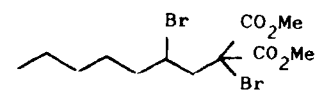 | 0,2 | (61) | 100 |
| 10 | $Br_2C(CO_2Me)_2$ (0,4) | 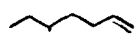 (0,2) | $Mn(OAc)_2$ (0,04) | $CNCH_2CO_2Me$ (0,04) | AcOH | AcOK (0,4) | 60 | 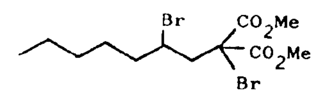 | 0,3 | (50) | 100 |
| 11 | $Br_2C(CO_2Me)_2$ (0,2) | 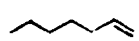 (0,4) | $Mn(OAc)_2$ (0,04) | $CNCH_2CO_2Me$ (0,04) | AcOH | AcOK (0,4) | 60 | 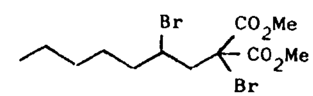 | 0,1 | (65) | 100 |
| 12 | $Br_2C(CO_2Me)_2$ (0,4) | 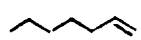 (0,4) | $Mn(OAc)_2$ (0,04) | $CNCH_2CO_2Me$ (0,04) | AcOH | AcOK (0,4) | 40 | 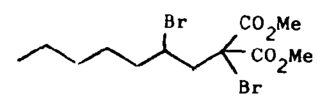 | 0,2 | (61) | 100 |

(a) Electricité consommée calculée par rapport à la concentration du réactif

(b) Rendement en produit isolé par rapport au réactif - Chiffres entre parenthèses = rendement chromatographique

(c) Taux de conversion du réactif

TABLEAU II (suite)

| Ex. No. | Réactif (mol.l$^{-1}$) | Substrat (mol.l$^{-1}$) | Médiateur (mol.l$^{-1}$) | Co-médiateur (mol.l$^{-1}$) | Solvant | Electrolyte support (mol.l$^{-1}$) | Temp. °C | Produit final | a F/mol | b ρ % | c t % |
|---|---|---|---|---|---|---|---|---|---|---|---|
| 13 | $Br_2C(CO_2Me)_2$ (0,4) | 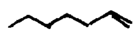 (0,4) | $Mn(OAc)_2$ (0,04) | $CNCH_2CO_2Me$ (0,04) | AcOH | AcOK (0,2) | 60 | 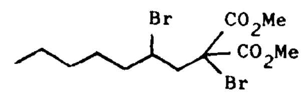 | 0,4 | (46) | 100 |
| 14 | $Br_2C(CO_2Me)_2$ (0,4) | 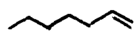 (0,4) | $Mn(OAc)_2$ (0,04) | $CNCH_2CO_2Me$ (0,04) | AcOH | $NEt_3$ (0,4) | 60 | 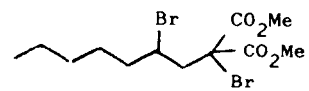 | 0,3 | (50) | 100 |
| 15 | $Br_2C(CO_2Me)_2$ (0,4) | 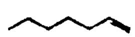 (0,4) | $Mn(OAc)_2$ (0,04) | $CNCH_2CO_2Me$ (0,04) | MeOH | AcOK (0,4) | 40 | 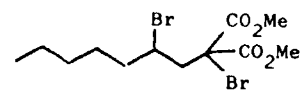 | 0,6 | (58) | 100 |
| 16 | $Br_2C(CO_2Me)_2$ (0,4) | 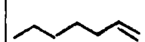 (0,4) | $Mn(OAc)_2$ (0,04) | $CNCH_2CO_2Me$ (0,04) | MeOH/ $H_2O$ (96:4) | AcOK (0,4) | 40 | 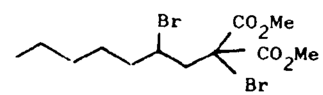 | 0,6 | (32) | 100 |
| 17 | $Br_2C(CO_2Me)_2$ (0,4) | 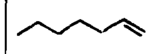 (0,4) | $Mn(OAc)_2$ (0,04) | $CNCH_2CO_2Me$ (0,04) | AcOH/ AcOEt (1:1) | AcOK (0,4) | 60 | 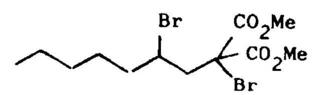 | 0,4 | (63) | 100 |

(a) Electricité consommée calculée par rapport à la concentration du réactif

(b) Rendement en produit isolé par rapport au réactif - Chiffres entre parenthèses = rendement chromatographique

(c) Taux de conversion du réactif

## EXEMPLES 18 à 29

Dans ces exemples, il est procédé de la manière indiquée à l'exemple 7 en ce qui concerne les conditions

générales.

On utilise toujours le cyanoacétate de méthyle en tant que co-médiateur mais, cette fois-ci, on fait varier la nature du réactif et du substrat.

On a réuni dans le tableau III ci-après la nature des réactifs mis en oeuvre, celle des substrats, celle des produits obtenus ainsi que, comme dans les autres cas, la quantité d'électricité consommée, le rendement et le taux de conversion du réactif.

TABLEAU III

| Ex. No. | Réactif ($mol.l^{-1}$) | Substrat ($mol.l^{-1}$) | Médiateur ($mol.l^{-1}$) | Co-médiateur ($mol.l^{-1}$) | Solvant | Electrolyte support ($mol.l^{-1}$) | Temp. °C | Produit final | a F/mol | b ρ % | c t % |
|---|---|---|---|---|---|---|---|---|---|---|---|
| 18 | $Br_2C(CO_2Me)_2$ (0,4) | 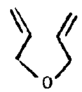 (0,8) | $Mn(OAc)_2$ (0,04) | $CNCH_2CO_2Me$ (0,04) | AcOH | AcOK (0,8) | 60 | 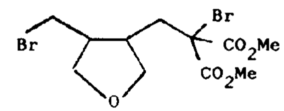 | 0,1 | 40 | 100 |
| 19 | $Br_2CHCO_2Me$ (0,4) | 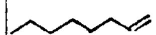 (0,8) | $Mn(OAc)_2$ (0,04) | $CNCH_2CO_2Me$ (0,04) | AcOH | AcOK (0,8) | 60 | 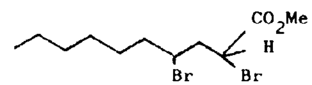 | 0,15 | 52 | 100 |
| 20 | $CBr_4$ (0,4) | 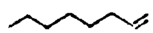 (0,8) | $Mn(OAc)_2$ (0,04) | $CNCH_2CO_2Me$ (0,04) | AcOH | AcOK (0,8) | 60 | 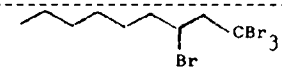 | 0,1 | 95 | 100 |
| 21 | $CBr_4$ (0,4) | 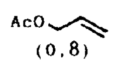 (0,8) | $Mn(OAc)_2$ (0,04) | $CNCH_2CO_2Me$ (0,04) | AcOH | AcOK (0,8) | 60 | 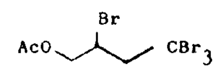 | 0,1 | 96 | 100 |
| 22 | $CBr_4$ (0,4) | 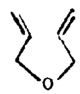 (0,8) | $Mn(OAc)_2$ (0,04) | $CNCH_2CO_2Me$ (0,04) | AcOH | AcOK (0,8) | 60 | 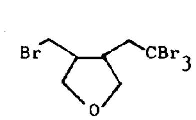 | 0,05 | 72 | 100 |
| 23 | $CBr_4$ (0,4) | 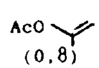 (0,8) | $Mn(OAc)_2$ (0,04) | $CNCH_2CO_2Me$ (0,04) | AcOH | AcOK (0,8) | 60 | $CH_3-\overset{O}{\overset{\|}{C}}-CH=CBr_2$ | 0,4 | 48 | 100 |

(a) Electricité consommée calculée par rapport à la concentration du réactif

(b) Rendement en produit isolé par rapport au réactif

(c) Taux de conversion du réactif

TABLEAU III (suite)

| Ex. No. | Réactif ($mol.l^{-1}$) | Substrat ($mol.l^{-1}$) | Médiateur ($mol.l^{-1}$) | Co-médiateur ($mol.l^{-1}$) | Solvant | Electrolyte support ($mol.l^{-1}$) | Temp. °C | Produit final | a F/mol | b ρ % | c t % |
|---|---|---|---|---|---|---|---|---|---|---|---|
| 24 | $CBr_4$ (0,4) | 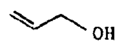 (0,8) | $Mn(OAc)_2$ (0,04) | $CNCH_2CO_2Me$ (0,04) | AcOH | AcOK (0,8) | 60 | 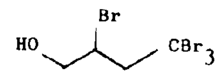 | 0,3 | 60 | 100 |
| 25 | $BrCCl_3$ (0,4) | 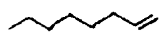 (0,8) | $Mn(OAc)_2$ (0,04) | $CNCH_2CO_2Me$ (0,04) | AcOH | AcOK (0,8) | 60 | 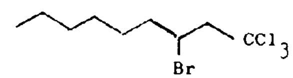 | 0,1 | 95 | 100 |
| 26 | $BrCCl_3$ (0,4) | 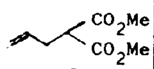 (0,8) | $Mn(OAc)_2$ (0,04) | $CNCH_2CO_2Me$ (0,04) | AcOH | AcOK (0,8) | 60 | 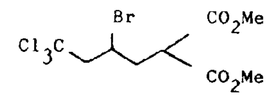 | 0,5 | 60 | 100 |
| 27 | $BrCCl_3$ (0,4) | 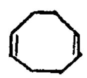 (0,8) | $Mn(OAc)_2$ (0,04) | $CNCH_2CO_2Me$ (0,04) | AcOH | AcOK (0,8) | 60 | 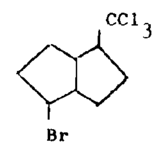 | 0,6 | 83 | 100 |
| 28 | $C_8F_{17}I$ (0,4) | 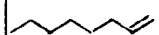 (0,8) | $Mn(OAc)_2$ (0,04) | $CNCH_2CO_2Me$ (0,04) | AcOH | AcOK (0,8) | T.A. | $CH_3(CH_2)_5CH(I)-(CF_2)_7CF_3$ | 0,1 | 80 | 100 |
| 29 | $CF_2Br_2$ (>1M) | 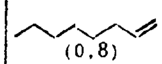 (0,8) | $Mn(OAc)_2$ (0,04) | $CNCH_2CO_2Me$ (0,04) | AcOH | AcOK (0,8) | T.A. | 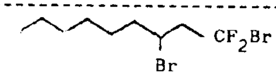 | 0.17 | 40 | n.d |

(a) Electricité consommée calculée par rapport à la concentration du réactif
(b) Rendement en produit isolé par rapport au réactif
(c) Taux de conversion du réactif
T.A. Température ambiante
n.d non déterminé.

## EXEMPLES 30 à 33

Ces exemples qui reprennent les conditions opératoires indiquées à propos de l'exemple 7, sont relatifs à l'addition de divers réactifs sur divers substrats acétyléniques.

Le co-médiateur mis en oeuvre est toujours le cyanoacétate de méthyle.

Les conditions opératoires, l'identification du réactif, du substrat et du produit obtenu ainsi que la quantité d'électricité consommée, le rendement et le taux de conversion du réactif correspondant à chaque réaction, sont réunis dans le tableau IV ci-après.

TABLEAU IV

| Ex. No. | Réactif (mol.l$^{-1}$) | Substrat (mol.l$^{-1}$) | Médiateur (mol.l$^{-1}$) | Co-médiateur (mol.l$^{-1}$) | Solvant | Electrolyte support (mol.l$^{-1}$) | Temp. °C | Produit final | a F/mol | b ρ % | c t % |
|---|---|---|---|---|---|---|---|---|---|---|---|
| 30 | $CBr_4$ (0,4) | 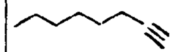 (0,8) | $Mn(OAc)_2$ (0,04) | $CNCH_2CO_2Me$ (0,04) | AcOH | AcOK (0,8) | 60 | 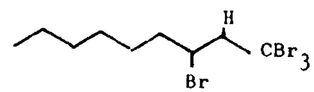 | 0,3 | 25 | 100 |
| 31 | $CBr_4$ (0,4) | Ph-C≡C-H (0,4) | $Mn(OAc)_2$ (0,04) | $CNCH_2CO_2Me$ (0,04) | AcOH | AcOK (0,8) | 60 | 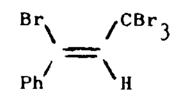 | 0,8 | 65 | 100 |
| 32 | $CF_2Br_2$ (>1M) | 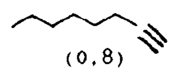 (0,8) | $Mn(OAc)_2$ (0,04) | $CNCH_2CO_2Me$ (0,04) | AcOH | AcOK (0,8) | T.A. | 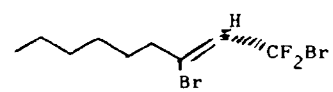 | 0,9 | 25* | n.d |
| 33 | $C_8F_{17}I$ (0,4) | 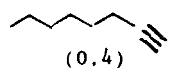 (0,4) | $Mn(OAc)_2$ (0,04) | $CNCH_2CO_2Me$ (0,04) | AcOH | AcOK (0,2) | T.A. | 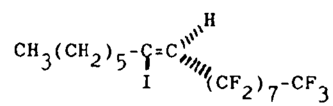 | 0,1 | 85* | 100 |

(a) Electricité consommée calculée par rapport à la concentration du réactif
(b) Rendement en produit isolé par rapport au réactif
(c) Taux de conversion du réactif
T.A. Température ambiante
n.d non déterminé
* somme des isomères E et Z avec Z ≫ E (ou Z/E > 10)

De l'ensemble des exemples qui précèdent, il apparaît que le procédé conforme à l'invention permet d'obtenir de nombreux composés polyfonctionnels dans des conditions qui, par rapport à celles des procédés existant déjà pour la préparation des composés en question, se distinguent par:

- une réalisation des réactions à des températures voisines de l'ambiante,
- une réalisation des réactions en cellule non divisée,
- une utilisation de quantités catalytiques du médiateur,
- une généralisation du procédé en ce que ce dernier ne nécessite pas que le réactif soit directement oxydable par le médiateur,
- une utilisation du réactif et du substrat en des quantités voisines des quantités stoechiométriques.

Ainsi, le produit d'addition de $CBr_4$ , $BrCCl_3$ ou $CF_2Br_2$ sur une oléfine permet l'accès à des diènes gem-dihalogénés.

A titre d'exemple, on indique la réaction:

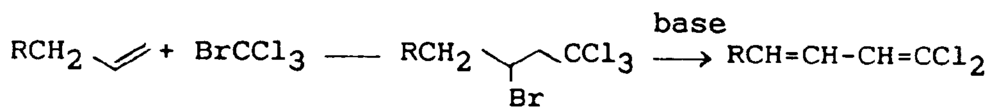


Les produits du type de celui ainsi obtenu, à savoir le $RCH=CH-CH=CCl_2$, peuvent être représentés par la formule

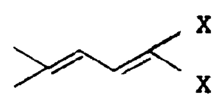

dans laquelle X représente Cl, Br, F, et qui constituent des précurseurs de pyréthroïdes connus pour leurs propriétés insecticides.

Les produits d'addition du bromomalonate de diméthyle peuvent être hydrolysés en γ-butyrolactone par la voie A (qui fournit des produits pharmaceutiques) ou en dérivé d'acide cyclopropane carboxylique par la voie B (qui fournit l'acide chrysanthémique ou analogues qui sont des insecticides).

Les voies A et B sont comme suit:

voie A 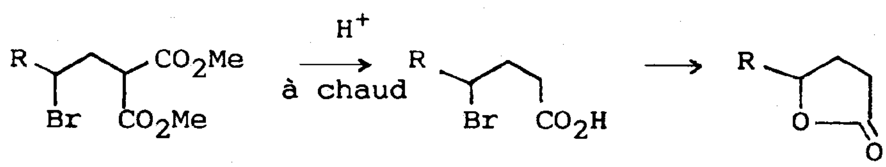


voie B 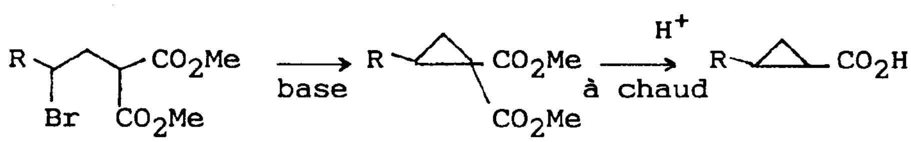


Le produit d'addition de $X_2CY_2$ sur l'acétate d'allyle permet l'accès à un cyclopropyl méthanol par la réaction suivante:

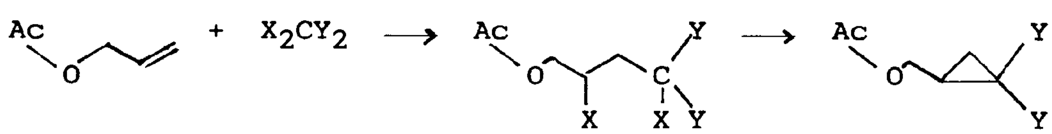

Ce produit est utilisé dans la préparation de médicaments comme, par exemple, le Bétaxolol III.

Les composés obtenus selon les exemples 2, 4 à 6, 18, 21 et 22 constituent des produits chimiques nouveaux.

En suite de quoi, on dispose d'un procédé électrochimique pour la préparation de composés organiques par voie radicalaire dont les caractéristiques résultent suffisamment de ce qui précède pour qu'il soit inutile d'insister à ce sujet et qui présentent, par rapport à ceux qui existent déjà, de nombreux avantages, à savoir

notamment celui de n'entraîner qu'une consommation d'électricité réduite et d'être facilement transposables à l'échelle industrielle.

## Revendications

1. Procédé électrochimique de préparation de composés organiques par voie radicalaire --suivant lequel on fait réagir un composé organique appelé "substrat" et présentant au moins une insaturation carbone-carbone avec un composé organique fonctionnalisé appelé "réactif" pour relier le substrat au réactif par une liaison carbone-carbone qui est créée par une réaction radicalaire amorcée par un métal appelé "médiateur" porté électrochimiquement et in situ à un degré d'oxydation élevé au sein d'un solvant polaire qui est compatible avec le potentiel d'oxydation du médiateur, qui contient un électrolyte-support et dans lequel plongent deux électrodes entre lesquelles on établit une différence de potentiel suffisante pour assurer l'oxydation électrochimique du médiateur-- est caractérisé par le fait que le réactif est choisi parmi ceux du groupe comprenant:
   - d'une part, les dérivés organiques halogénés oxydables comportant un atome d'hydrogène énolisable de formule

   $$XCHA_2$$

   dans laquelle:
      - X est un atome d'halogène du groupe Cl, Br, I et
      - A est un radical du groupe -CN, $-COR'$ et $-CO_2R'$ avec R′ représentant un radical alkyle fonctionnalisé ou non, linéaire ou ramifié, notamment les radicaux méthyle, éthyle et tertio-butyle,
   - d'autre part, les dérivés organiques halogénés non oxydables ne comportant pas d'hydrogène énolisable et répondant aux formules:

   $$X_2CY_2 \text{ et}$$
   $$X_2CA_2$$

   dans lesquelles X et A ont les significations indiquées ci-dessus et Y représente un atome de chlore ou de fluor, étant entendu que ces dérivés organiques halogénés non oxydables sont mis en oeuvre en présence d'un composé à méthylène actif choisi dans le groupe comprenant ceux des formules:

   $$CNCH_2CO_2R'$$
   $$MeCOCH_2CO_2R'$$
   $$MeCOCH_2COMe$$

   dans lesquelles Me est le radical méthyle et R′ a les significations indiquées plus haut.

2. Procédé selon la revendication 1, caractérisé par le fait que le solvant polaire est choisi dans le groupe comprenant l'acide acétique, le méthanol et l'acétonitrile et parmi les mélanges d'un solvant polaire protique et d'un solvant polaire aprotique.

3. Procédé selon l'une des revendications 1 et 2, caractérisé par le fait que l'électrolyte support est un acétate de sodium, de potassium ou d'ammonium, présent à une concentration d'environ 0,1 à environ 1, de préférence de 0,4 à 0,8 mol/litre.

4. Procédé selon l'une des revendications 1 à 3, caractérisé par le fait que le substrat insaturé est choisi parmi
   - les alcènes ou cyclo-alcènes fonctionnalisés ou non,
   - les diènes conjugués ou non,
   - les alcynes fonctionnalisés ou non.

5. Procédé selon l'une des revendications 1 à 4, caractérisé par le fait que les concentrations en substrat et en réactif sont toutes deux comprises entre environ 0,1 et environ 1,5, de préférence entre 0,4 et 1 mol/litre et que le rapport des concentrations en substrat et en réactif est compris entre environ 0,5 et environ 5 et, de préférence, entre 0,1 et 2.

6. Procédé selon l'une des revendications 1 à 5, caractérisé par le fait que le médiateur est choisi dans le groupe comprenant le manganèse (III), le cobalt (III), le cérium (IV) et le fer (III) et qu'il est mis en oeuvre à une concentration d'environ 0,01 à environ 0,1, de préférence comprise entre 0,02 et 0,04 mol/litre.

7. Composé nouveau susceptible d'être obtenu par le procédé selon l'une des revendications 1 à 6, présentant la formule:

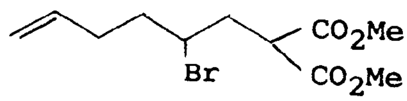
.

8. Composé nouveau susceptible d'être obtenu par le procédé selon l'une des revendications 1 à 6, présentant la formule:

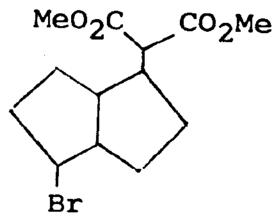
.

9. Composé nouveau susceptible d'être obtenu par le procédé selon l'une des revendications 1 à 6, présentant la formule:

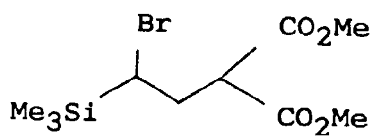

10. Composé nouveau susceptible d'être obtenu par le procédé selon l'une des revendications 1 à 6, présentant la formule:

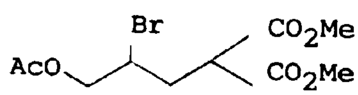
.

11. Composé nouveau susceptible d'être obtenu par le procédé selon l'une des revendications 1 à 6, présentant la formule:

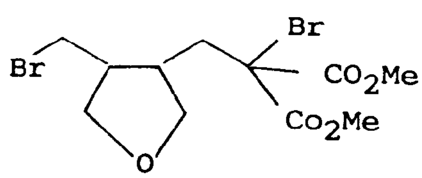
.

12. Composé nouveau susceptible d'être obtenu par le procédé selon l'une des revendications 1 à 6, présentant la formule:

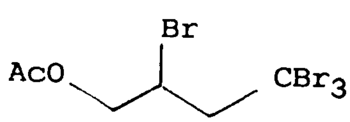
.

13. Composé nouveau susceptible d'être obtenu par le procédé selon l'une des revendications 1 à 6, présentant la formule:

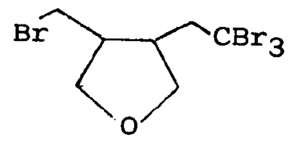

.

Office européen des brevets

# RAPPORT DE RECHERCHE EUROPEENNE

Numero de la demande

EP 92 40 1167
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | GB-A-1 017 978 (PITTSBURGH PLATE GLASS)<br>* exemple III *<br>--- | 12 | C07B37/02<br>C07C69/65<br>C07C69/635<br>C07C69/675<br>C07D307/10<br>C07D307/16<br>C07F7/08<br>C07C67/347<br>C25B3/10 |
| D,A | TETRAHEDRON, (INCL. TETRAHEDRON REPORTS)<br>vol. 47, no. 4/5, 1991, OXFORD GB<br>pages 831 - 840;<br>R. SHUNDO ET AL: 'CARBON-CARBON BOND FORMATION USING MANGANESE(III) ACETATE AS AN ELECTROCHEMICAL MEDIATOR'<br>* le document en entier *<br>--- | 1-6 | |
| A | BULLETIN OF THE ACADEMY OF SCIENCES OF THE USSR, DIVISION OF CHEMICAL SCIENCE<br>vol. 34, no. 10, 20 Avril 1986, NEW YORK US<br>pages 2223 - 2224;<br>R. A. AMRIEV ET AL: 'ADDITION OF DIETHYL BROMOMALONATE AND DIETHYL DIBROMOMALONATE TO VINYLTRIMETHYLSILANE'<br>* le document en entier *<br>--- | 1-6 | |
| A | JOURNAL OF THE CHEMICAL SOCIETY, SECTION B PHYSICAL ORGANIC CHEMISTRY<br>1967, LETCHWORTH GB<br>pages 803 - 805;<br>J. I. G. CADOGAN ET AL: 'SYNTHETIC ASPECTS OF FREE-RADICAL ADDITION REACTIONS. PART VI. THE RELATIVE REACTIVITIES OF ETHYL MALONATE AND RELATED COMPOUNDS TOWARDS OCT-1 ENE'<br>* le document en entier *<br>--- | 1-6 | DOMAINES TECHNIQUES RECHERCHES (Int. Cl.5)<br>C07B<br>C07C<br>C25B<br>C07D |
| A | SYNTHESIS.<br>no. 3, Mars 1977, STUTTGART DE<br>pages 145 - 154;<br>R. FREIDLINA ET AL: 'SYNTHETIC APPLICATIONS OF HOMOLYTIC ADDITION AND TELOMERISATION REACTIONS OF BROMINE-CONTAINING ADDENDS WITH UNSATURATED COMPOUNDS CONTAINING ELECTRON-WITHDRAWING SUBSTITUENTS'<br>* page 149 - page 154 *<br>--- | 1-6 | |

Le présent rapport a été établi pour toutes les revendications

| Lieu de la recherche | Date d'achèvement de la recherche | Examinateur |
|---|---|---|
| LA HAYE | 14 AOUT 1992 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 92 40 1167
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| P,X | CHEMICAL ABSTRACTS, vol. 115, no. 23, 9 Décembre 1991, Columbus, Ohio, US; abstract no. 255280, J. Y. NEDELEC & K. NOHAIR: 'MANGANESE(III)-PROMOTED FREE-RADICAL ADDITION OF DIMETHYL BROMOMALONATE TO OLEFINS USING AN ELECTROCHEMICAL REGENERATION PROCEDURE.' page 779 ;colonne 2 ; & SYNLETT 1991, No. 9, Pages 659-660 * abrégé * ----- | 1-6 | |

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| LA HAYE | 14 AOUT 1992 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)